Europäisches Patentamt

European Patent Office

Office européen des brevets

(11) Veröffentlichungsnummer: **0 305 842**
**A2**

(12) **EUROPÄISCHE PATENTANMELDUNG**

(21) Anmeldenummer: 88113609.7

(22) Anmeldetag: 22.08.88

(51) Int. Cl.⁴: **C08K 5/09 , C08K 5/00 , C08L 23/02 , A61L 15/07**

(30) Priorität: 02.09.87 DE 3729262

(43) Veröffentlichungstag der Anmeldung:
08.03.89 Patentblatt 89/10

(84) Benannte Vertragsstaaten:
AT BE CH DE ES FR GB GR IT LI LU NL SE

(71) Anmelder: **BAYER AG**
**Konzernverwaltung RP Patentabteilung**
**D-5090 Leverkusen 1 Bayerwerk(DE)**

(72) Erfinder: **von Bonin, Wulf, Dr.**
**Mendelssohnstrasse 30**
**D-5090 Leverkusen(DE)**
Erfinder: **von Gizycki, Ulrich, Dr.**
**Wiembachallee 24**
**D-5090 Leverkusen 3(DE)**

(54) **Thermoplastisches Konstruktionsmaterial.**

(57) Thermoplastisches Konstruktionsmaterial enthält einen Träger und eine Polymerzubereitung, die aus einem schmelzbaren Polymerisat, einer kristallisierenden Carbonsäure oder einem Carbonsäurederivat und gegebenenfalls aus Kristallisationsmodifikatoren besteht. Das thermoplastische Konstruktionsmaterial kann besonders für Stützverbände verwendet werden.

EP 0 305 842 A2

## Thermoplastisches Konstruktionsmaterial

Die Erfindung betrifft thermoplastisches Konstruktionsmaterial, insbesondere für medizinische Stützverbände oder technische Vorrichtungen, ein Verfahren zu seiner Herstellung und seine Verwendung.

Das erfindungsgemäße Konstruktionsmaterial besteht im allgemeinen aus einem Träger, der mit einer Polymerzubereitung beschichtet und/oder imprägniert ist, und gegebenenfalls weiteren Füll- oder Hilfsstoffen.

Im allgemeinen können die erfindungsgemäßen Konstruktionsmaterialien zur Versteifung, Formgebung und Abdichtung im medizinischen oder technischen Bereich verwendet werden.

Die erfindungsgemäßen Konstruktionsmaterialien können aber auch zur Herstellung von Behältern, Filtern, von Rohren, zum Verbinden von Konstruktionselementen, zur Fabrikation von dekorativen oder künstlerischen Artikeln, zu Versteifungszwecken oder als Füll- bzw. Dichtungsmaterial für Fugen und Hohlräume eingesetzt werden.

Medizinische Stützverbände auf Basis von hydraulisch abbindenden Materialien, z.B. als Gipsverband, sind bekannt.

Stützverbände, die wesentlich leichter und röntgendurchlässiger sind, benutzen organische Reaktivharze, zumeist auf Isocyanatbasis, die ähnlich wie Gipsbinden unter Wasserzusatz aushärten (DE-A-23 57 931).

In beiden Fällen muß mit Wasser, zumindest in Form von Luftfeuchtigkeit, gearbeitet werden.

Es ist auch bekannt, Platten aus Polycaprolacton, Guttapercha oder trans-Polybutadien bei Temperaturen unter 100 °C zu Verformen und nach Erkalten und Erstarren als medizinische Stützvorrichtung zu verwenden.

Diese Formkörper sind nicht geeignet, Gegenstände mit konischen Erhebungen bzw. variablen Radien, z.B. einem menschlichen Bein oder ein Gelenk, durch Umwinden mit Binden unter schlüssiger Haftung der einzelnen Bindenlagen untereinander zu stützen.

Es wurde ein thermoplastisches Konstruktionsmaterial enthaltend eine Polymerzubereitung und einen Träger gefunden, das dadurch gekennzeichnet ist, daß die Polymerzubereitung aus
- einem schmelzbaren Polymerisat mit einer Glasübergangstemperatur unter 150 °C,
- einer kristallisierenden Carbonsäure und/oder einem Carbonsäurederivat und
- gegebenenfalls Kristallisationsmodifikatoren besteht.

Überraschenderweise erhält man durch die erfindungsgemäße Zusammensetzung der Polymerzubereitung ein Material, das in einem für die Anwendung geeigneten Temperaturbereich (beispielsweise bei Temperaturen über 30 °C, bevorzugt 40 bis 100 °C) eine mäßig klebende, dünnflüssige Schmelze (eine sogenannte unterkühlte Schmelze) bildet, die nach dem Auskristallisieren eine steife und feste Masse bildet. Die steife und feste Masse erweicht erst bei Temperaturen über 40 °C, bevorzugt im Bereich von 45 bis 70 °C. Die erfindungsgemäßen thermoplastischen Konstruktionsmaterialien lassen sich daher ohne Beeinträchtigung durch höhere Temperaturen auch im medizinischen Bereich anwenden.

Als schmelzbares Polymerisat mit einer Glasübergangstemperatur (TG) unter 150 °C kommen solche Polymerisate in Betracht, die bei 10 bis 40 °C amorph, semikristallin, teilkristallin oder flüssig-kristallin sind, und nach DIN 53 735 Schmelzindices ($MiF_2$) von 1,5 bis 30, vorzugsweise 5 bis 15, aufweisen. Hierbei kann es sich um Polymere beliebiger sterischer Konfiguration oder Taktizität handeln. Bevorzugt werden Polymerisate die zumindest teilweise kristallin sind.

Als schmelzbare Polymerisate seien bevorzugt genannt: Homopolymere oder Copolymere von Mono- und/oder Diolefinen mit den möglichen cis- oder trans-Konfigurationen, Taktizitäten oder sonstigen sterischen Isomeren. Beispielsweise seien hier Homo- und Copolymere von Ethylen, Propylen, Butenen, Pentenen, Hexenen und höheren geradkettigen oder verzweigten Olefinen ($C_2$-$C_{18}$) genannt, z.B. Polyethylen, Ethylen-Propylen-Copolymere, Polypropylen, Polyisobutylen, Polypenten, Polymethylpenten, Polyocten, Polyoctadecen. Als Comonomere kommen neben den genannten Olefinen beispielsweise in Betracht: Vinylester wie Vinylacetat, Vinylversat, Vinylether, Acrylsäure, Methacrylsäure, Maleinsäure, Itaconsäure sowie deren Ester mit $C_1$-$C_{24}$-Alkoholen, wie Methanol, Ethanol, Butanol, Dodecanol oder Octadecanol, oder ungesättigte Nitrile wie (Meth)Acrylnitril, ungesättigte Alkohole, wie Allylalkohol, (Meth)Acrylamid, Cyanacrylester, wie α-Cyanacrylsäure-methyl, -ethyl, -propyl, -butyl, -dodecylester, Vinylaromaten, wie (Methyl)-Styrol.

Außerdem seien Homopolymerisate und/oder Copolymerisate von Diolefinen (unabhängig von der Taktizität und der sterischen Konfiguration) wie Naturkautschuk, Guttapercha, diisomeren Polybutadiene, Polyisoprene, Polycylopentene, Polycyclooctene, sogenannte Polyoctenamere, Polycyclopentadien, Norbornenpolymerisate, Cyclopentadienpolymerisate, Polychloroprene, Polydimethylbutadiene oder Olefinpolymere genannt, wie sie durch radikalische oder ionische Polymerisation oder durch Ziegler-Natta-Katalyse oder auch sonstigen, z.B. bei der Crackerraffination von Erdöl anfallenden Olefinen und Olefingemischen, erhalten werden können.

Als schmelzbare Polymerisate seien auch aliphatische oder aromatische Polyamide, wie Perlon- oder Nylon-Typen, Polystyrole, Polyester z.B. auf Caprolactonbasis oder auf (Tere)phthalsäure-, Adipinsäurebasis, Polyvinylchlorid, Polycarbonate, Polyether, Polyacetale, Polysulfide, Polysulfone sowie Mischformen und Abmischungen solcher Polymere genannt.

Solche Polymeren haben Molgewichte über 10.000, vorzugsweise über 30.000.

Insbesondere bevorzugt sind die Homo- und Copolymerisate von Ethylen und Propylen. Insbesondere seien hier radikalisch erhaltene Ethylen- Homo- und Copolymerisate mit Acrylestern ($C_1$-$C_{18}$) oder Vinylestern ($C_1$-$C_{22}$) genannt, enthaltend 10-85, vorzugsweise 25-55 Gew.-% cis-Vinylacetat Esteranteil.

Erfindungsgemäße kristallisierende Carbonsäuren und/oder Carbonsäurederivate sind im allgemeinen löslich oder dispergierbar in dem schmelzbaren Polymerisat. Bevorzugt werden Typen bzw. Kombinationen solcher Typen ohne Mischungslükken im Polymerisat bei 100 - 170°C.

Der Schmelzpunkt der erfindungsgemäßen kristallisierenden Carbonsäuren und/oder Carbonsäurederivate liegt im allgemeinen im Bereich von 40 bis 115°C, bevorzugt von 55 bis 95°C.

Erfindungsgemäße kristallisierende Carbonsäuren und/oder Carbonsäurederivate sind im allgemeinen Mono- oder Polycarbonsäuren, bevorzugt Mono- oder Dicarbonsäuren, mit 12 bis 100, bevorzugt 18 bis 60, Kohlenstoffatomen. Beispielsweise seien Naphthensäuren oder Terpensäuren, wie die Abietinsäure, Montansäure, durch Paraffinoxydation zugängliche Carbonsäuren, Bernsteinsäure, Maleinsäure, Phthalsäure, Adipinsäure, Azelainsäure und die Alkylbernsteinsäure genannt. Als Derivate seien Anhydride, Ester($C_1$-$C_{35}$), Amide und Salze genannt, wobei Ester bevorzugt werden.

Besonders werden hier gesättigte Fettsäuren bzw. diese enthaltende Fettsäuregemische bzw. deren Ester oder Salze oder Amide mit Schmelzbereichen zwischen 35 und 95°C, vorzugsweise von 50 bis 85°C, genannt. Als Salze kommen vor allem solche von 2- und 3-wertigen Metallen wie Calcium, Magnesium, Zink, Aluminium oder von Aminen mit mehr als 9 Kohlenstoffatomen bzw. deren unter 100°C schmelzende Gemische in Betracht. Als Amide seien Derivate genannt, die von Ammoniak, Mono- oder Dialkanolaminen oder aliphatischen, cylcoaliphatischen, araliphatischen, aromatischen Aminen mit 1 bis 66 Kohlenstoffatomen abgeleitet werden können.

Als Ester kommen insbesondere auch Fettsäureglyceride oder Phospholipide in Betracht, sowie Ester mit Polyalkoholen wie Sorbit, Pentaerythrit, Zucker, Trimethylolpropan, Glykolen, Alkanolaminen, Methanol oder kristallisierenden Fettalkoholen mit 12 bis 28 Kohlenstoffatomen.

Als Fettsäure werden vorzugsweise Montansäure, Laurinsäure, Myristinsäure, Palmitinsäure, Stearinsäure und Behensäure bzw. diese enthaltende natürliche und raffi nierte oder hydrierte bzw. synthetische Gemische zugrundegelegt. Besonders bevorzugt ist die Verwendung von Stearinsäure und deren Derivaten, z.B. Glyzerindistearat, Montanwachs, Bienenwachs, Wollfett, Kollophonium.

Kristallisationsmodifikatoren sind im allgemeinen Verbindungen mit einem Molekulargewicht unter etwa 10 000, die vorzugsweise in dem Gemisch aus dem schmelzbaren Polymerisat und der kristallisierenden Carbonsäure bzw. dem Carbonsäurederivat bei 100 - 170°C löslich sind. Der Schmelzpunkt des Kristallisationsmodifikators liegt im allgemeinen im Bereich von -60 bis +160°C, bevorzugt von 5 bis 100°C, insbesondere 50 - 110°C.

Die erfindungsgemäßen Kristallisationsmodifikatoren sind vorzugsweise Paraffine, Paraffinöle, Paraffinweichwachse und -hartwachse, Montan- und Pflanzenwachse, bzw. Oligomere aus den auf Seite 4 beispielhaft genannten Monomeren bzw. deren Gemische. Es kommen aber auch in Betracht oligomere Ester und/oder Amide von Abietinsäure oder Terpensäuren, Naphthensäuren oder Gemischen dieser Säuren. Es seien auch Lanolin, Petroleumharze, Raffineriewachse sowie aliphatische, araliphatische, cyclische oder aromatische Polyether wie Phenylethylenoligomere, Polyethylenglykole, Polypropylenglykole, Additionsprodukte von Ethylen- und/oder Propylenoxid an mono- und/oder polyfunktionelle Alkohole mit 1 bis 28 Kohlenstoffatomen und Polytetrahydrofuranharze genannt. Es können auch oligomere Homo- und Copolymerisate von Fettalkoholestern bzw. Fettamin-basierten Amiden ungesättigter Carbonsäuren wie (Meth)Acrylsäure, Malein-, Fumar- oder Itaconsäure mit Ethylen, Isobutylen oder Styrol verwendet werden.

Ebenfalls in Betracht kommen Produkte, wie sie durch Oligomerisierung von Olefinen oder z.B. oxidativen Abbau von Olefinpolymerisaten erhalten werden. Beispielsweise seien Ethylenpolymerisate (Polyethylenwachse) genannt.

Die erfindungsgemäßen Polymerzubereitungen enthalten im allgemeinen 20 bis 70 Gew.-Teile, bevorzugt 30 bis 60 Gew.-Teile, des schmelzbaren Polymerisats, 15 bis 80 Gew.-Teile, bevorzugt 20 bis 65 Gew.-Teile der kristallisierenden Carbonsäuren und/oder des Carbonsäurederivats und 0 bis 50 Gew.-Teile, bevorzugt 5 bis 35 Gew.-Teile des Kristallisationsmodifikators. Die Prozentangaben addieren sich im Einzelfall natürlich zu 100 %.

Als Füllstoffe kommen anorganische und organische Produkte in Form von Pulvern, Perlen, Hohlperlen, Plättchen oder Fasern in Betracht. Beispielsweise seien Polyolefinpulver, z.B. aus Niederdruckpolyethylen oder -propylen, Formaldehydharz auf Phenol- oder Amidbasis, Kohlenstoffpulver, Holzmehle, Stärke, Cellulose und Fruchtkernmehle genannt. Als anorganische Füllstoffe kommen Siliciumdioxid oder Oxide, Hydroxide, Phosphate, Sulfate, Carbonate und Silikate von vorzugsweise 2- und 3-wertigen Metallen in Betracht, wie Magnesium, Calcium, Zink, Barium, Aluminium und Eisen.

Bevorzugt werden Kieselsäurefüllstoffe und/oder Polyolefinpulver mit Korndurchmessern unter 25 $\mu$m, vorzugsweise unter 1,5 $\mu$m.

Der Füllstoff wird im allgemeinen in einer Menge von 0 bis 75 Gew.-Teile, bevorzugt von 0 bis 25 Gew.-Teile, eingesetzt.

Weiterhin ist es möglich, Hilfsstoffe den thermoplastischen Konstruktionsmaterialien zuzugeben. Beispielsweise seien anorganische oder organische Farbpigmente und Farbstoffe, z.B. auf Titan- oder Eisenoxidbasis oder auf Basis fettlöslicher organischer Farbstoffe, Geruchs- und Aromastoffe, Detergentien zur Beeinflussung von Wasseraufnahme und Reinigungsfähigkeit, rheologische Fließverbesserer, z.B. auf Basis polarer Hochpolymere oder Siliconöle, Fungizide, Bakterizide, Stabilisatoren gegen Umwelteinflüsse, Markierungsmittel, phobierende oberflächenverändende Zusätze auf Basis von Polysiloxanen oder Polyfluorverbindungen genannt.

Die erfindungsgemäßen thermoplastischen Konstruktionsmaterialien enthalten im allgemeinen die Hilfsmittel im Bereich von 0 bis 5 Gew.-Teilen, bevorzugt von 0 bis 3 Gew.-Teilen.

Als Träger werden im allgemeinen anorganische oder organische Substrate verwendet die über 100 $^\circ$C schmelzen, formstabil und mechanisch belastbar sind.

Träger können beispielsweise Folien oder Bändchen, z.B. aus Metallen oder Kunststoffen sein, die gegebenenfalls geschlitzt oder gelocht sind, um Netze, Fasern, Faserbündel, Garne, Drähte, insbesondere aber um Papiere, Vliese, Gelege, Geflechte, Gestricke, Gewebe oder Gewirke, bzw. deren Mischformen aus mineralischen und/oder organischen Fasern zu bilden. Es seien Fasern aus Kaolin, Aluminiumoxiden, Siliciumdioxid, Glasfasern der verschiedensten Art, z.B. sogenanntes R-Glas oder S-Glas, Metallen (z.B. Eisen, Aluminium oder Kupfer), Kohlenstofffasern, natürliche oder synthetische Fasern aus Baumwolle, Seide, Zelluloseabkömmlinge, nachbehandelte und modifizierte Zellulosen, Collagene, Caseine oder sonstige Proteine, aliphatische oder aromatische Polyimide, Polyamide, Polyester, Polyurethane, Polyether, Polyacrylnitriltypen und Polyolefinfasern in Betracht. Selbstverständlich sind auch Mischfasern möglich.

Die Fasern können in verschiedenen, vorzugsweise flächigen Geometrien oder Mustern angeordnet sein. Insbesondere bevorzugt werden Binden von 2 bis 25 cm Breite von Gelegen, Geweben, Gewirken aus Zellulosefasern, Baumwollgarn, Polyester-, PAN-, Polyolefin-, Polyamidgarn, Glasfasern und deren Mischungen. Insbesonders werden Glas-, Baumwoll- und Polyester-Trägermaterialien verwendet, beispielsweise Glasfasergewirke, bestehend aus Glasfasergarnen mit einem Elastizitätsmodul >7.000 daN/mm$^2$, einer Maschenlochzahl von 3 bis 30 pro cm$^2$ einer Längendehnung bis 50 %, einer Querdehnbarkeit bis 100 % und einem m$^2$-Gewicht ungedehnt von 200 bis 900 g.

Es kommen aber außer Binden auch Schnüre, Fäden oder nicht endlose Bahnen, bzw. Tücher oder Matten von verschiedensten Formaten in Betracht.

Es wurde auch ein Verfahren zur Herstellung der erfindungsgemäßen thermoplastischen Konstruktionsmaterialien gefunden, dadurch gekennzeichnet, daß man eine Polymerzubereitung aus einem schmelzbaren Polymerisat mit einer Glasübergangstemperatur unter 150 $^\circ$C, einer kristallisierenden Carbonsäure oder Carbonsäurederivat und gegebenenfalls einem Kristallisationsmodifikator durch Mischen herstellt, gegebenenfalls weitere Füll- und Hilfsstoffe zugibt und damit den Träger beschichtet und/oder imprägniert.

Zur Herstellung der erfindungsgemäßen Konstruktionsmaterialien wird der Träger mit einer im Bereich von 50 bis 1500, vorzugsweise 200 bis 800, g/m$^2$ liegenden Beaufschlagsmenge an zuvor auf 80 bis 200 $^\circ$C, vorzugsweise 100 bis 150 $^\circ$C vorgeheizter Schmelze aus dem gut gerührten Gemisch der einzelnen Komponenten beaufschlagt.

Die geschmolzene Zubereitung kann vorzugsweise eine homogene einphasige Lösung oder aber auch mehrphasig dispers sein. Es wird zweckmäßigerweise unter Luftausschluß, z.B. unter Stickstoff oder CO$_2$ als Schutzgas, gearbeitet.

Der Träger wird mit der Polymerzubereitung getränkt, imprägniert oder beschichtet. Vorzugsweise heizt man den Träger bei der Imprägnierung auf die Schmelztemperatur auf.

Die Beaufschlagung kann auch durch Vereinigung von Substrat und Ausrüstung durch Erhitzen gemeinsam im Verpackungsbehälter erfolgen.

Die Beaufschlagung kann allseitig und durchdringend oder sandwichartig oder einseitig, teildurchtränkt oder partiell und in Form bestimmter durch die spätere Verwendung vorgegebenen Mustern, z.B. Punkten, Streifen usw. erfolgen.

Die Beaufschlagung erfolgt z.B. auf beheizbaren Walzstühlen, Rakelanlagen, Tränkbädern, Sprühanlagen, Siebdruckanlagen oder Extrusionsanlagen.

Zur Lagerung kann das beaufschlagte Substrat vor, während oder nach dem Erstarren in eine z.B. gefaltete oder gewickelte Lagerform gebracht werden. Zweckmäßigerweise wird es durch Tüten oder Dosen vor Zutritt von Feuchte, Frischluft und Licht geschützt. Die Aufbewahrung erfolgt bevorzugt unter Schutzgasen wie Stickstoff oder Kohlendioxid.

Vor der Verwendung muß das Versteifungsmaterial auf dem Substrat aufgeschmolzen worden sein. Das wird erreicht, z.B. indem man die Verwendung unmittelbar an den Beaufschlagungsprozeß für das Substrat anschließt, z.B. das Substrat, etwa eine Binde, durch einen mit Abstreifer versehenen Vorrat an Schmelze zieht und dann sofort wickelt. Es ist jedoch auch möglich, das vorzeitig ausgerüstete Material durch Vorweglagerung in einem Wärmeschrank in offener oder aufgerollter Form zweckmäßigerweise unter Schutzgas aufzuschmelzen und dann aus dem Wärmeschrank zur sofortigen Applikation im aufgeschmolzenen Zustand gegebenenfalls auch aus dem entsprechenden Behälter zu entnehmen.

Es ist auch beispielsweise möglich, daß man eine z.B. bei 100 bis 150° C wieder aufgeschmolzene Binde über eine Kühlzone im Temperaturbereich 35 bis 70° C führt und dann appliziert.

Die Wärmezufuhr kann durch Wärmekontaktflächen aus Teflon, sonstigen Polymeren, Metall, Glas, Keramik, durch Dampf, Wasser, Heißluft oder Mikrowellen, Hochfrequenz oder IR-Strahler erfolgen. Man kann eine solche Binde, z.B. auch erst unmittelbar vor der Applikationsstelle durch Kontaktflächen, mittels eines Heißluftstroms oder durch einen IR-Strahler aufschmelzen bis Flexibilität erreicht ist und so applizieren.

Die erfindungsgemäßen thermoplastischen Konstruktionsmaterialien werden bevorzugt als technisches Konstruktionsmaterial und für medizinische Zwecke als Verbandsmaterial mit Stützfunktion eingesetzt.

Das geschieht z.B. indem der zu stützende Bereich nach der Medikation, gegebenenfalls mit einer zusätzlichen Wärme-isolierenden Schicht aus Watte, Verbandsmaterial oder Schaumstoff abgedeckt und dann mit dem erfindungsgemäßen, z.B. in Form einer Bandage vorliegenden Stützverbandsmaterial umwickelt wird. Die Zahl der aufzubringenden Schichten bestimmt die zu erzielende Festigkeit und Steifigkeit bei gegebenen Materialtypen, und kann wunschgemäß gewählt werden.

Durch gegebenenfalls partielles Erwärmen mit Warmluft kann das Material jederzeit nachgerichtet oder nachmodelliert werden. Es ist nach Gebrauch auch bei RT abwickelbar und kann gegebenenfalls wiederverwendet werden.

Im orthopädischen Bereich und in der Tiermedizin kann das erfindungsgemäße thermoplastische Konstruktionsmaterial auch zur Herstellung von Schutz- und Stützvorrichtungen verwendet werden.

Im technischen Bereich können die erfindungsgemäßen Materialien im Modellbau, als Material zur Herstellung von Abformungen, Abdrücken, zu Versteifungszwecken, zu Verbindungs- und Fixierungszwecken und als Dichtungsmaterial oder als Halbzeug, das durch thermoplastische Formpressen oder Wickeln zu Formteilen verarbeitet worden ist bzw. werden kann, eingesetzt werden.

Beispiele

Beispiel 1

Es wird bei 190° C (N₂) eine Mischung hergestellt aus:
300 Teilen Stearinsäure
25 Teilen Paraffinwachs Fp. ca. 45° C
25 Teilen Paraffinwachs Fp. ca. 65° C
100 Teilen handelsüblichem Ethylenvinylacetat-Copolymerisat mit 45 % Vinylacetatgehalt (Levapren 450, BAYER AG)
50 Teilen Ethylen-Homopolymerisat mit einem Schmelzindex gemäß DIN 53 735 von ca. 7 (Baylon 19 N, BAYER AG).

Diese Mischung wird gut gerührt und dann nach Ausbildung einer klaren Schmelze auf 150° C abgekühlt (Trübung bei 98° C, Erstarrung bei 51° C). Dann wird bei 150° C ein Glasfasergewirke (250 g/m²; Maschenweitenabstand (Schuß) 50, Maschenstababstand (Kette) 50 pro 10 cm; Längsdehnung 12 %, Querdehnung ca. 85 %) durch die Schmelze gezogen und über eine Folge von beheizten Umlenkstäben abgequetscht. Die Beaufschlagung mit der Schmelze beträgt 420 g/m².

Dann wird das in Form einer 10 cm breiten quasi endlosen Binde vorliegende, schmelzimprägnierte Material auf einen Dorn gewickelt und bei 110° C in einer Alu-Hülse gelagert.

Dann wird beispielhaft über einen Dorn von 3 cm Durchmesser eine anliegende Hülle aus Seidenpapier gelegt. Man entnimmt nun dem Wärmeschrank die 110° C heiße Aluhülse mit Schutzhandschuhen. Dann öffnet man die Hülse, entnimmt den geschmolzenen Wickel und rollt ihn teilweise auf. Das abgerollte Verbandteil kann bereits mit bloßen Händen um den mit Papier geschützten Dorn gelegt werden, da es schnell abkühlt. Es wird dann weiter zu einem achtlagigen Rollverband um den Dorn gewickelt und abgeschnitten. Die Lagen haften gut aneinander, neigen nicht zum Abgleiten. In 90 Sek. ist die Außenschicht soweit verfestigt, daß der Verband ohne zu kleben noch modelliert werden kann. Nach Maßgabe des Abkühlens kristallisiert der Verband aus und wird fest. Er erreicht seine Endfestigkeit bei 20° C nach ca. 15 bis 20 Minuten. Der Wickel wird 5 Min. nach Herstellung von dem Dorn gezogen. Nach 48 h wird er bei 20°C mit 100 kg auf Mantellinie des 10 cm langen und 3,6 cm dicken achtlagigen Zylinders mit 3 cm lichter Weite belastet. Es erfolgt kein Zusammenbrechen, sondern nur eine Verformung um 2 mm.

Beispiel 2

Die Mischung gemäß Beispiel 1 wird auf 150°C erhitzt und so geschmolzen. Bei dieser Temperatur wird mit der Mischung ein Baumwollgewebe in Leinenbindung 150 g/m², mit 215 g/m² Schmelze imprägniert.

Dann wird aus dem imprägnierten Material ein Streifen von 1 cm Breite und 2,7 cm Länge geschnitten. Der Streifen wird an einem Ende 1 cm tief in eine Holzklemme eingeklemmt und dann in einen gläsernen, doppelwandigen Isolierzylinder gehängt, so daß der 45 cm lange Zylinder mit 7 cm lichter Weite den Streifen nach oben und unten je zu etwa 10 cm Länge überragt.

Dann wird mit 4,3 m³/h ein 150°C heißer Luftstrom von unten nach oben durch den senkrecht stehenden Zylinder geschickt. Dabei schmilzt die Imprägnierung des Streifens auf. Nach eben 1 Min. Heizzeit wird der Heißluftstrom gegen einen Warmluftstrom mit xx°C ausgetauscht. Dann wird die Zeit gemessen, die bei der Temperatur xx°C vergeht, bis der imprägnierte Streifen fest ist.

Diese Zeit ist ein Maß für die Unterkühlbarkeit der Schmelze bzw. die Kristallisationsverzögerung bei der Temperatur xx°C. Diese Temperatur wird im Bereich zwischen 30 und 50°C gewählt, da in diesem Bereich etwa die Applizierbarkeit am Körper noch möglich ist ebenso wie die Handhabbarkeit mit ungeschützten Fingern.

Bei xx = 45°C wird für die Mischung gemäß Beispiel 1 (1) eine Flexibilitätszeit (F) von 185 Sek. gemessen, d.h. F = 185.
Führt man die Messung mit Stearinsäure anstatt des Gemisches (1) durch, so wird für (F) die Größe 14 gemessen.
Ersetzt man in Mischung (1) das EVac-Polymerisat durch ein gleichartiges Ethylen-Ethylacrylat-Copolymerisat, so hat (F) die Größe von 210.
Ersetzt man in Beispiel 1 die Stearinsäure durch Glycerin-Monostearat, so hat (F) die Größe 195.
Vertauscht man in (1) das Homo- und das Copolymerisat mengenmäßig, so hat (F) die Größe 110.
Läßt man in Mischung (1) die Paraffinwachse weg, so hat (F) die Größe 125.
Läßt man in Mischung (1) das EVac-Polymerisat weg, so hat (F) die Größe von 41.
Läßt man in Mischung (1) das Homopolymerisat weg, so hat (F) die Größe 161.

Beispiel 3

Analog Beispiel 1 wird eine 10 cm breite und 80 cm lange Binde aus dem Glasfasergewirke mit 550 g/m² der Mischung getränkt und eng aufgerollt, wobei ein Wickel von ca. 3 cm Durchmesser erhalten wird.

Dieser Wickel wird in eine Aluminiumhülse mit Deckel von ca. 3,3 cm lichter Weite und 10,5 cm lichter Höhe, die auf der Mantelfläche einen achsparallelen Schlitz besitzt (Breite ca. 1 mm, Länge ca. 100 mm), so eingelegt, daß wie bei einem Kleinbildfilm ein kleines Stück der Binde aus dem Schlitz herausragt. In der Dose wird der Wickel bei Raumtemperatur gelagert. Nach 40 Tagen wird die Dose mit Wickel in einen auf 130°C (N₂) eingestellten Wärmeschrank gebracht. Nach 2 h wird die Dose entnommen, dann in eine die Dose isolierende Hülse gesteckt, die einen seitlich zu ca. 25 % geöffneten, eng anliegenden Zylinder aus Holz oder Kunststoff darstellt. Aus der Öffnung läßt sich jetzt die Binde leicht herausziehen und fest um den gemäß Beispiel 1 verwendeten Dorn wickeln, ohne daß bei der Handhabung durch die erhitzte Binde Verbrennungen an den Händen des Anwenders auftreten. Nach Abkühlen hat dieser Wickel eine um ca. 15 kg verbesserte Festigkeit.

Beispiel 4

In einem mit Knetelementen ausgestatteten und variabel beheizbaren Extruder wird eine folgende Mischung zu einer homogenen Schmelze verarbeitet (130°C) und unter Kühlung als Strang ausgetragen:
100 Teile Ethylen-Vinylacetat-Copolymerisat, 45 % VAc-Gehalt,
100 Teile Stearinsäure und
100 Teile Paraffinwachs, Fp. ca. 70°C.
Der Strang wird granuliert.

In einem auf ca. 60°C aufgeheizten Extruder wird aus dem Granulat ein 10 cm breiter und ca. 1,5 mm dicker endloser Strang über eine Breitschlitzdüse ausgetragen.

Dieser Strang besteht aus zäher Schmelze und wird einseitig beim Austritt aus dem Extruder auf eine Binde aus Polyester-Wirkware von ebenfalls 10 cm Breite aufgelegt, diese läuft mit dem Extrusionsstrang mit und wird dann zu einem 2 m langen Wickel aufgerollt. Der Wickel wird in eine Hülse aus Niederdruckpolyethylen gegeben und so aufbewahrt. Etwa 24 h vor der Applikation wird eine solche Binde dann mit Hülse in einen auf 75°C eingestellten Heizschrank oder ein entsprechend temperiertes Bad gegeben.

Dann wird die Binde entnommen. Beim Ausrollen kühlt sie soweit ab, daß sie ohne Schwierigkeiten auch mit ungeschützten Händen schnell um das zu versteifende Gelenkteil, bzw. im Beispielfall den in Beispiel 1 benutzten Demonstrationsdorn gewickelt werden kann und dort unter Verklebung der Lagen zu erstarren beginnt. In abgekühltem Zustand erreicht der Wickel nach ca. 15 Min. seine Endfestigkeit.

Beispiel 5

Eine Mischung aus
100 Teilen Ethylenvinylacetat-Copolymerisat (gemäß Beispiel 1)
50 Teilen Ethylen-Homopolymerisat (gemäß Beispiel 1)
50 Teilen Paraffinwachs Fp. ca. 70°C
50 Teilen Glycerin-monostearat
wird bei 170°C unter $N_2$ intensiv homogenisiert, die Schmelze abgekühlt und nach Erstarrung homogenisiert.

Das Granulat wird auf einem Kalandar bei Walzentemperaturen um 35°C zu einer ca. 1,5 mm dicken Folie verarbeitet; aus dieser Folie werden 10 cm breite Bänder geschnitten, die eine Länge von ca. 200 cm haben. Diese Bänder werden zusammen mit einer etwa gleichlangen und ebenfalls 10 cm breiten Binde gemäß Beispiel 1 zu einem Wickel aufgerollt und in einer verdeckelten Aluminiumhülse aufbewahrt, die so eng ist, daß sich der enggerollte Wickel nicht aufrollen kann.

Vor der Applikation wird diese Dose dann ca. 2 h in einem 150°C heißen Temperierschrank getempert, wobei die Folie aufschmilzt und die umgebende Binde durchtränkt.

Dann wird auf 68°C heruntergekühlt, indem man die Dose nun in einem 68°C-Schrank lagert.

Bei Entnahme ist die Binde gut durchtränkt und ohne Schwierigkeiten gut als Versteifungsverband auf das durch eine Lage Zellstoff oder Watte oder einen Schutzverband geschützte Glied aufzubringen.

Ein zehnlagiger Wickel aus dieser Binde mit ca. 3 cm lichter Weite kann auf der Mantellinie mit ca. 100 kg belastet werden ohne bei 19°C zu brechen.

Beispiele 6 bis 10

Durch homogenes Vermischen mit einem Rührwerk unter $N_2$ wird bei 150 bis 170°C eine Schmelze aus den folgenden Komponenten hergestellt und mit 400 g/m² durch Schmelztauchbeschichtung auf eine 10 cm breite Binde aus einem Glasfasergewirke gemäß Beispiel 1 aufgebracht.

Diese so ausgerüstete Binde wird als 1 m langer Streifen über einen Dorn mit 1,1 cm Durchmesser aufgerollt. Der so erhaltene Wickel mit ca. 4 cm Durchmesser wird in Alufolie verpackt und in einem auf 68°C temperierten Heizschrank gelagert.

Bei dieser Temperatur kann der so vorkonditionierte Wickel mit den bloßen Händen gut gehandhabt werden.

Man entnimmt den temperierten Wickel dem Heizschrank, entfernt die Alufolie und kann jetzt sofort mit dem Umwickeln des zu schützenden Gliedes bzw. Körperteils beginnen, ohne Belästigungen durch Wärme befürchten zu müssen.

Hier wird beispielhaft wiederum mit dem 1-m-Stück Binde der auch in Beispiel 1 verwendete Dorn stramm umwickelt. Nach jeder Wickellage findet durch die anhaltende Klebrigkeit der erfindungsgemäßen Systeme eine Verklebung der Lagen statt, so daß letztlich ein sehr stabiler Wickel erhalten wird.

Beispiel 6 bis 16

Jeder der gemäß den folgenden Beispielen im Analogium Beispiel 5 hergestellten 1-m-Wickel hält bei Raumtemperatur eine Belastung von ca. 100 kg gemäß Beispiel 1 aus, ohne zu brechen.

Beispiel 6

100 Gew.-Teile Ethylen-Vinylacetat-Copolymerisat,
100 Gew.-Teile Stearinsäure
100 Gew.-Teile Paraffinwachs Fp. ca. 71°C

Beispiel 7

50 Gew.-Teile Ethylen-Vinylacetat-Copolymerisat,
50 Gew.-Teile Octenamer-Polymerisat Fp. ca. 60°C (Polycyclooten)
100 Gew.-Teile Stearinsäure
100 Gew.-Teile Paraffinwachs Fp. ca. 71°C

Beispiel 8

100 Gew.-Teile Octenamer-Polymerisat Fp. ca. 60°C (Polycyclooten)
25 Gew.-Teile Stearinsäure

Beispiel 9

100 Gew.-Teile Octenamer-Polymerisat Fp. ca.
60°C (Polycyclooten)
20 Gew.-Teile Stearinsäure
10 Gew.-Teile Glycerin-monostearat

Beispiel 10

100 Gew.-Teile Octenamer-Polymerisat Fp. ca.
60°C (Polycyclooten)
25 Gew.-Teile Paraffinwachs Fp. ca. 71°C
25 Gew.-Teile Paraffinwachs Fp. ca. 63°C

Beispiel 11

100 Gew.-Teile Ethylen-Vinylacetat-Copolymerisat,
100 Gew.-Teile Stearinsäure
50 Gew.-Teile Paraffinwachs Fp. ca. 71°C
50 Gew.-Teile Polyethylen

Beispiel 12

100 Gew.-Teile Ethylen-Vinylacetat-Copolymerisat,
100 Gew.-Teile Stearinsäure
50 Gew.-Teile Paraffinwachs Fp. ca. 71°C
50 Gew.-Teile Hartparaffin mit einem Erstarrungspunkt ca. 97°C
50 Gew.-Teile Polyethylen

Beispiel 13

100 Gew.-Teile techn. Behensäure, ca. 85 % C-22
100 Gew.-Teile Paraffinwachs Fp. ca. 63°C
100 Gew.-Teile Ethylen-Vinylacetat-Copolymerisat

Beispiel 14

300 Gew.-Teile techn. Behensäure, ca. 85 % C-22
50 Gew.-Teile Paraffinwachs Fp. ca. 71°C
100 Gew.-Teile Ethylen-Vinylacetat-Copolymerisat,
50 Gew.-Teile Polyethylen

Beispiel 15

75 Gew.-Teile Glycerin-monostearat
25 Gew.-Teile Montansäure
100 Gew.-Teile Paraffinwachs Fp. ca. 71°C
100 Gew.-Teile Ethylen-Vinylacetat-Copolymerisat

Beispiel 16

200 Gew.-Teile Stearinsäure
100 Gew.-Teile Montansäure
50 Gew.-Teile Paraffinwachs Fp. ca. 71°C
100 Gew.-Teile Ethylen-Vinylacetat-Copolymerisat,
50 gew.-Teile Polyethylen

Beispiel 17

50 Gew.-Teile Hochdruckpolyethylen, 100
Gew.-Teile Ethylen-Vinylacetat-Copolymer, 300
Gew.-Teile Stearinsäure werden als homogene
Schmelze zum Tränken einer Binde aus Polyester-
Baumwoll-Mischgewebe (65:35) in Leinenbindung
mit einem Flächengewicht von 350 g/m² bei 125°C
eingesetzt. Die Auflagemenge der wachsartigen Mischung beträgt 330 g/m². Der Penetrometerwert
nach Klein bei 45°C ist 6-7, bei 21°C 2,0. Aus der
10 cm breiten und 1 m langen Binde wird ein
Wickel gedreht und bei 68°C gelagert, nachdem er
in Aluminiumfolie eingeschlagen worden ist.

Nach 24 h Lagerzeit wird der Wickel entnommen und gemäß Beispiel 1 um einen Musterdorn
gewickelt.

Der Wickel ist 10-lagig und kann achsparallel bei
21°C mit ca. 100 Kg Auflagegewicht belastet werden ohne zusammenzubrechen.

**Ansprüche**

1.  Thermoplastisches Konstruktionsmaterial
enthaltend eine Polymerzubereitung und einen Träger, dadurch gekennzeichnet, daß die Polymerzubereitung aus
- einem schmelzbaren Polymerisat mit einer Glasübergangstemperatur unter 150°C,
- einer kristallisierenden Carbonsäure oder Carbonsäurederivat und
- gegebenenfalls einem Kristallisationsmodifikator
besteht.

2.  Thermoplastisches Konstruktionsmaterial
nach Anspruch 1, dadurch gekennzeichnet, daß
das schmelzbare Polymerisat Schmelzindices
[MiF₂] von 1,5 bis 30 aufweist.

3. Thermoplastisches Konstruktionsmaterial nach Anspruch 1, dadurch gekennzeichnet, daß als schmelzbares Polymerisat Homo- oder Copolymere von Olefinen eingesetzt werden.

4. Thermoplastisches Konstruktionsmaterial nach den Ansprüchen 1 bis 3, dadurch gekennzeichnet, daß als schmelzbares Polymerisat Homo- oder Copolymere von $C_2$-$C_6$-Olefinen eingesetzt werden.

5. Thermoplastisches Konstruktionsmaterial nach den Ansprüchen 1 bis 4, dadurch gekennzeichnet, daß als schmelzbares Polymerisat Homopolymere des Ethylens oder Propylens eingesetzt werden.

6. Thermoplastisches Konstruktionsmaterial nach den Ansprüchen 1 bis 4, dadurch gekennzeichnet, daß als schmelzbares Polymerisat Copolymere des Ethylens und/oder Propylens mit Acrylaten oder Vinylestern eingesetzt werden.

7. Thermoplastisches Konstruktionsmaterial nach den Ansprüchen 1 bis 6, dadurch gekennzeichnet, daß als kristallisierende Carbonsäure oder Carbonsäurederivat Verbindungen mit einem Schmelzpunkt im Bereich von 40 bis 100 °C eingesetzt werden.

8. Thermoplastisches Konstruktionsmaterial nach den Ansprüchen 1 bis 7, dadurch gekennzeichnet, daß kristallisierende Carbonsäuren oder Carbonsäurederivate eingesetzt werden, die sich in dem schmelzbaren Polymerisat lösen.

9. Thermoplastisches Konstruktionsmaterial nach den Ansprüchen 1 bis 8, dadurch gekennzeichnet, daß als kristallisierende Carbonsäuren oder Carbonsäurederivate gesättigte Fettsäuren, deren Ester oder Amide bzw. Gemische dieser Komponenten eingesetzt werden.

10. Thermoplastisches Konstruktionsmaterial nach den Ansprüchen 1 bis 9, dadurch gekennzeichnet, daß als Kristallisationsmodifikatoren Verbindungen im Schmelzbereich von -60 bis +120 °C eingesetzt werden.

11. Thermoplastisches Konstruktionsmaterial nach den Ansprüchen 1 bis 10, dadurch gekennzeichnet, daß als Kristallisationsinhibitoren Paraffine eingesetzt werden.

12. Thermoplastisches Konstruktionsmaterial nach den Ansprüchen 1 bis 11, dadurch gekennzeichnet, daß die Polymerzubereitung,
20 bis 70 Gew.-Teile des schmelzbaren Polymerisats,
15 bis 80 Gew.-Teile der kristallisierenden Carbonsäure oder des Carbonsäurederivats und 0 bis 50 Gew.-Teile des Kristallisationsinhibitors enthält.

13. Verfahren zur Herstellung von thermoplastischem Konstruktionsmaterial, dadurch gekennzeichnet, daß man eine Polymerzubereitung aus
- einem schmelzbaren Polymerisat mit einer Glasübergangstemperatur unter 150 °C,
- einer kristallisierenden Carbonsäure oder eines Carbonsäurederivats und
- gegebenenfalls eines Kristallisationsmodifkators herstellt, gegebenenfalls weitere Füll- und Hilfsstoffe zugibt und damit den Träger beschichtet und/oder imprägniert.

14. Verwendung einer Polymerzubereitung aus
- einem schmelzbaren Polymerisat mit einer Glasübergangstemperatur unter 140 °C,
- einer kristallisierenden Carbonsäure oder eines Carbonsäurederivats und
- gegebenenfalls eines Kristallisationsinhibitors und gegebenenfalls weiterer Füll- oder Hilfsstoffen, aufgebracht auf ein Trägersubstrat als Konstruktionsmaterial.

15. Verwendung nach Anspruch 14 als medizinische Stützverbände.